# FASCICULE DE BREVET EUROPEEN

(11) **EP 2 186 539 B1**
(45) Date de publication et mention de la délivrance du brevet: **18.06.2014**
(21) Numéro de dépôt: 08356142.3
(22) Date de dépôt: 17.11.2008
(51) Int. Cl.: A61M 16/06, A61M 16/08, A61M 16/10

(54) **Embout spécifique pour masque à oxygène "Haute Concentration"**
Spezialanschluss für Maske zur Versorgung mit hochkonzentriertem Sauerstoff
Specific nozzle for a High-Concentration Oxygen mask.

(43) Date de publication de la demande: 19.05.2010
(73) Titulaire: Wasylkiewicz, Jacek, 69500 Bron (FR)
(72) Inventeur: Wasylkiewicz, Jacek, 69500 Bron (FR)

(56) Documents cités:
- EP-A- 0 911 051
- FR-A- 2 873 042
- US-A- 3 960 148
- US-A- 5 492 114
- US-A- 5 701 886

## Description

La présente invention concerne un embout spécifique, **qui s'adapte sur un** masque à Oxygène "haute concentration" **en** ventilation spontanée, **et permettant une captation optimale du CO2 expiré, grâce** à un système de prélèvement du CO2 expiré pour permettre **1'** analyse **de ses caractéristiques** séparément du O2 inspiré. Elle concerne également un masque à Oxygène "haute concentration" **en ventilation spontanée** équipé d'un tel embout, ainsi qu'un ensemble de surveillance respiratoire comprenant un tel masque.

Un masque à Oxygène "haute concentration" **en ventilation spontanée** est un masque simple (à usage unique) qui se compose, de façon classique, d'un corps souple, réalisé par exemple en poly-chlorure de vinyle (PVC). Ce corps, qui recouvre approximativement en service la bouche et le nez du patient, reçoit un embout qui est notamment solidarisé par coopération de formes. Cet embout est muni d'un conduit d'arrivée d'Oxygène, de forme appropriée, permettant l'alimentation du patient, ainsi que d'une poche en plastique souple qui sert de réservoir pour assurer la concentration d'Oxygène et permettre l'augmentation du flux pendant la phase d'inspiration du malade. Ce masque est donc destiné à l'oxygénation d'un patient qui respire spontanément, et non pas à "l'assistance respiratoire" qui se fait avec un masque plus complexe, étanche, non à usage unique, et raccordé à des moyens de régulation de l'Oxygène fourni.

Un masque à Oxygène "haute concentration" **en ventilation spontanée,** du type décrit ci-dessus, trouve son application dans de nombreuses circonstances.

Ainsi, il est tout d'abord utilisé dans la réanimation des malades, en salle de réveil post-opératoire, aux soins intensifs, ou dans les cas d'urgence en tous lieux, notamment en réanimation dans tous les cas de détresse respiratoire. Il est également possible de faire appel à un tel masque dans différentes pathologies respiratoires nécessitant un apport en Oxygène sans pression mais très concentrée, et une surveillance respiratoire par capnomonitorage. Enfin, on tire parti de ce masque à Oxygène "haute concentration" **en ventilation spontanée** dans le cadre d'interventions chirurgicales, qui sont réalisées **sans assistance respiratoire.**

On conçoit que l'alimentation en Oxygène par l'intermédiaire de ce masque s'accompagne d'une surveillance respiratoire du patient. Ceci permet de s'affranchir de tout risque d'accident, susceptible de se révéler dramatique.

Dans cette optique, il est tout d'abord connu de contrôler le taux de saturation du sang en Oxygène, par exemple par un capteur infrarouge placé au bout du doigt du patient. Cette solution ne s'avère cependant pas suffisamment réactive, en particulier en cas d'une baisse sensible de la fréquence respiratoire du patient.

II est également possible de mettre en oeuvre cette surveillance respiratoire, par le personnel soignant lui-même. Cependant, cette solution alternative est peu satisfaisante, en termes de sécurité. De plus, elle nécessite l'emploi d'une main d'oeuvre importante et relativement qualifiée, qui n'est pas toujours disponible notamment lors de soins effectués dans des véhicules ou hors des hôpitaux, pour des interventions d'urgence sur le terrain où ces masques "haute concentration" **en ventilation spontanée** sont particulièrement utilisés.

Ceci étant précisé, l'invention se propose de remédier à l'ensemble des inconvénients de l'art antérieur évoqués ci-dessus pour les masques "haute concentration" **en ventilation spontanée.**

**L'état de la technique actuelle et des brevets existants fait ressortir les documents suivants :**

Le document brevet US-A-5 701 886 décrit un système (masque avec embout) destiné à délivrer de l'**Oxygène** et un médicament aérosol (par nébulisation) inhalé par le patient. L'administration du médicament aérosol se fait au travers du raccord 50 (voir sens de la flèche sur la figure 1 de ce document), qui ne sert donc pas au prélèvement du gaz expiré comme dans la présente invention, mais seulement à mesurer la dose de médicament insufflée.

Le critère de nouveauté de la nouvelle invention réside essentiellement dans le mode de captation du CO² dans les gaz expirés par le patient, pour permettre de mesurer capnographiquement la fréquence et la capacité respiratoire de celui-ci. Ce CO² est capté dans les masques à **Oxygène non étanches,** de façon à l'isoler par rapport à l'Oxygène présent dans ces masques. Il s'agit là de masques simples (à usage unique) qui fournissent simplement de l'Oxygène (sans pression, mais concentré) pour aider les malades à mieux s'oxygéner notamment en phase de réveil post-opératoire ou en réanimation dans tous les cas de détresse respiratoire. On appelle cela des patients sous "ventilation spontanée". Mais les masques ne comportent classiquement aucun moyen intégré de mesure efficace du gaz expiré.

Les masques "haute concentration" comportent classiquement une poche réservoir pour maintenir la capacité d'Oxygène disponible. Ils diffèrent donc des masques normaux, dits "moyenne concentration", qui n'ont pas cette poche.

Pour les masques "moyenne concentration", le même inventeur a déjà déposé un brevet (brevet **d'invention FRANCE** FR-A-2 873 042 **et brevet Européen** EP 1 618 912**),** pour l'embout spécifique à ces masques et répondant aux mêmes objectifs que cette présente invention (captation optimale du CO² expiré).

Le document brevet US-A-3 960 148 décrit un appareil (masque avec embout) destiné à l'anesthésie générale (par administration d'un gaz anesthésique, contrôlé par débitmètre). Ceci s'applique donc à des masques **étanches** qui permettent une adaptation exacte au visage du patient pour ne pas avoir de fuites du produit anesthésiant. Dans ce document, le prélèvement par la connexion 19 du gaz expiré (qui comporte de l'anesthésiant) est simplement évacué par une pompe.

Le document brevet EP-A-0 911 051 décrit un embout destiné à l'assistance respiratoire, c'est-à-dire (selon le [0001] ligne 1-1 de ce document) pour "des patients dont la respiration spontanée est absente ou insuffisante". Le gaz est "injecté" et le système décrit par ce document est destiné à mesurer la pression et la composition du gaz expiré.

Le document brevet US-A-5 492 114 décrit un système spécifique de protection du manchon d'entrée du réservoir d'**Oxygène** pour les masques à Oxygène haute concentration.

**La présente invention telle que revendiquée a donc** pour objet particulièrement un embout spécifique **qui s'adapte sur un masque** à Oxygène "haute concentration" **en ventilation spontanée, et permettant une captation optimale du CO2 expiré,** propre à être solidarisé par rapport à un corps de ce masque, cet embout comprenant des moyens de connexion, propres à être raccordés à des moyens de fourniture d'Oxygène, ainsi qu'à une poche en plastique souple qui sert de réservoir pour assurer la concentration d'Oxygène. Il est **caractérisé en ce que** cet embout comprend surtout des moyens efficaces de prélèvement du CO2 expiré par le patient, ces moyens de prélèvement étant propres à être raccordés à un appareil (type Capnomètre) de détermination d'au moins un paramètre respiratoire du patient (analyse par monitorage capnographique). Ces moyens spécifiques, sont beaucoup plus complexes que ceux qui peuvent être utilisés sur des masques (non usage uniques) étanches, car le CO2 expiré dans les masques usage unique est rejeté directement du masque à l'extérieur, et le prélèvement efficace du CO2 nécessite une adaptation très précise du capteur pour que le prélèvement soit significatif. Ceci a donc nécessité de nombreuses recherches et expérimentations pour aboutir aux embouts objet de ce brevet.

Selon d'autres caractéristiques de l'invention :
- les moyens de connexion et les moyens de prélèvement s'étendent à partir d'une embase dudit embout, cette embase étant propre à venir en appui contre un siège du corps du masque ;
- les moyens de prélèvement comprennent une chambre de prélèvement, s'étendant à partir de l'embase en direction d'un volume intérieur du corps (coté masque), ainsi qu'un raccord de prélèvement extérieur s'étendant à partir de l'embase à l'opposé dudit volume intérieur, ce raccord de prélèvement débouchant directement dans la chambre de prélèvement ;
- l'embout comprend un tube propre à prendre appui contre l'embase du corps, et faisant saillie en direction du volume intérieur vers le masque, saillie de ***dimension appropriée*** pour permettre le bon prélèvement du CO2 expiré dans la chambre autour de ce tube ;
- à l'opposé du volume intérieur, à partir de l'embase, une chambre d'alimentation (indépendant de la chambre de prélèvement) s'étendant à partir de l'embase en direction d'un volume extérieur du corps (coté poche réservoir en plastique souple), ainsi qu'un conduit de connexion aux dits moyens de fourniture d'Oxygène, ce conduit de connexion débouchant directement dans la chambre d'alimentation ;
- cette chambre d'alimentation est en communication directe avec la chambre de prélèvement (vers le masque) mais séparé par une valve anti-retour ;
- la partie extérieure de cette chambre d'alimentation, est propre à venir s'insérer dans la poche réservoir en plastique souple, par tout moyen approprié.

L'invention a également pour objet l'ensemble d'un masque à Oxygène "haute concentration" **en ventilation spontanée,** comprenant un corps destiné à recouvrir le nez et la bouche d'un patient, ainsi qu'un embout propre à être solidarisé par rapport au corps, notamment par emmanchement, **caractérisé en ce que** l'embout est tel que défini ci-dessus.

L'invention ne se limite pas à l'adaptation d'un tel embout suivant le premier type (A) selon figures 1 et 2. A noter que ce premier type (A) a fait l'objet de la demande de Brevet d'invention FRANCE N° 0705882000 du 17/08/2007, dont la présente invention représente sa demande d'extension européenne. Mais l'invention porte également sur une variante qui est le deuxième type (B) selon figures 3 et 4 qui diffère du premier type en ce qu'il comporte dans l'embout la valve d'expiration (destinée à évacuer le reste des gaz expirés, non prélevés par le système de monitorage capnographique) intégrée dans l'embout, au lieu des valves habituellement intégrées dans les joues latérales du corps du masque.

L'invention a enfin également pour objet un ensemble de surveillance respiratoire comprenant :
- un masque à Oxygène "haute concentration" **en ventilation spontanée,** qui est tel que défini ci-dessus ;
- des moyens de fourniture d'Oxygène ;
- des moyens de raccordement entre ces moyens de fourniture d'Oxygène et les moyens de connexion appartenant au masque ;
- des moyens de détermination d'au moins un paramètre respiratoire du patient, et
- des moyens de raccordement entre ces moyens de détermination et les moyens de prélèvement appartenant au masque.

Selon d'autres caractéristiques de l'invention :
- cet ensemble peut comprendre également une alarme propre à être activée si une valeur du ou de chaque paramètre respiratoire, déterminée par les moyens de détermination, se situe en dehors d'une plage prédéterminée ;
- un des paramètres respiratoire est la fréquence respiratoire du patient ;
- un autre paramètre respiratoire est le taux de dioxyde de carbone présent dans le gaz expiré par le patient.

L'invention sera mieux comprise et d'autres avantages de celle-ci apparaîtront plus clairement à la lumière de la description gui va suivre d'un ensemble de surveillance respiratoire conforme à son principe, donnée uniquement à titre d'exemple non limitatif et faite en référence aux dessins annexés, dans lesquels :

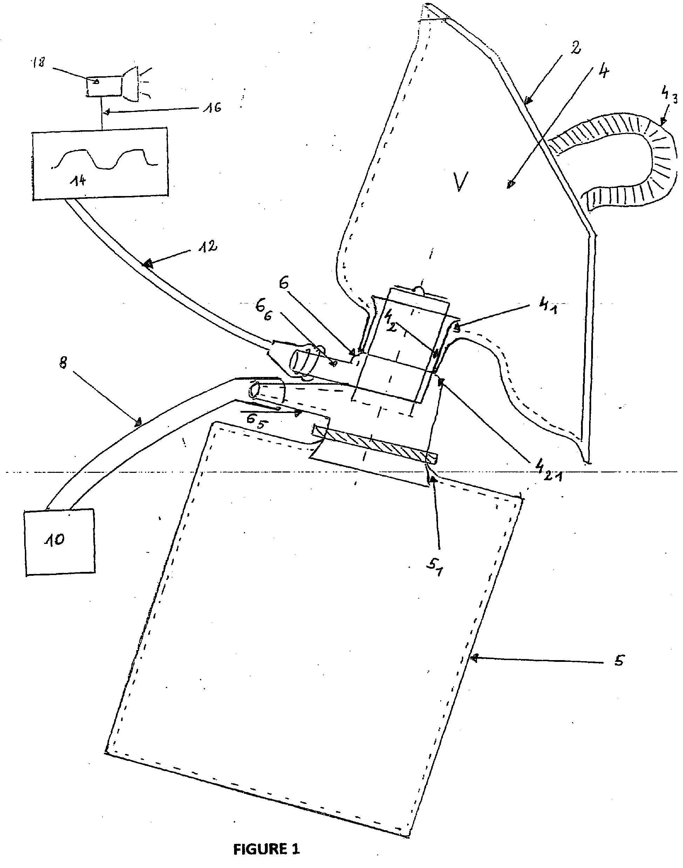

- la figure 1 est une vue schématique, illustrant un ensemble de surveillance respiratoire conforme à l'intention : embout du premier type (A) ;

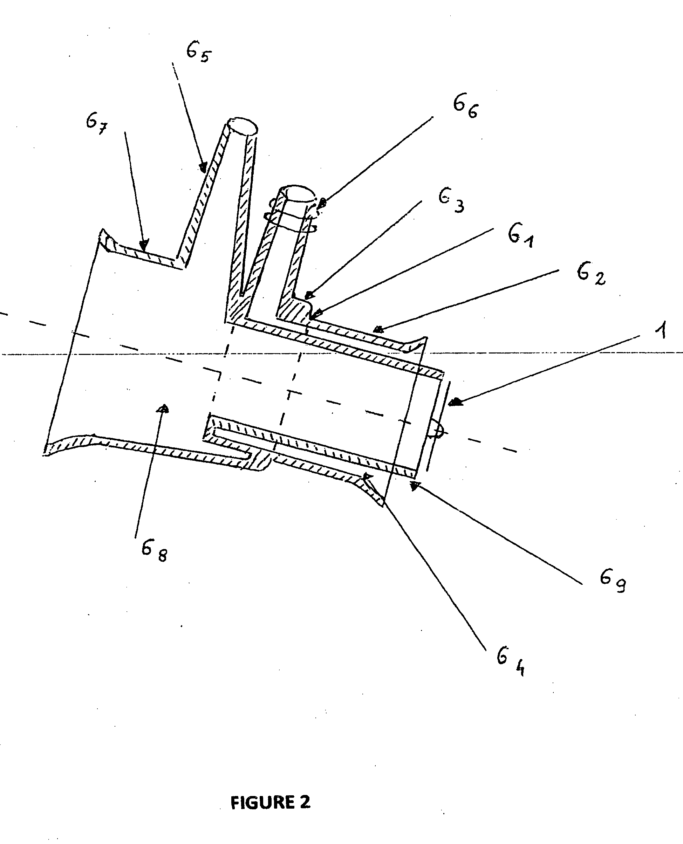

- la figure 2 est une vue en coupe, de côté, à plus grande échelle, illustrant un embout du premier type (A) appartenant à un masque à Oxygène "haute concentration" **en ventilation spontanée** qui équipe l'ensemble de surveillance respiratoire de la figure 1.

L'ensemble de surveillance respiratoire, représenté sur la figure 1, comprend tout d'abord un masque à Oxygène "haute concentration" **en ventilation spontanée,** désigné dans son ensemble par la référence 2.

Ce dernier comporte, de façon connue en soi, un corps souple 4, réalisé notamment en PVC (ou poly-chlorure de vinyle). Ce corps souple 4 est destiné à recouvrir, en service, le nez et la bouche du patient, sans que l'étanchéité soit parfaitement assurée.

Dans sa partie antérieure, en référence à sa position portée par le patient, le corps souple est creusé d'un orifice 4₁, à partir duquel s'étend un col 4₂, dirigé à l'opposé du volume intérieur V du masque. Comme on le verra de façon plus détaillée dans ce qui suit, ce col 4₂ est destiné à la réception d'un embout 6.

Enfin, le corps 4 est pourvu d'un lien élastique 4₃, propre à être disposé autour de la tête du patient, afin d'assurer la tenue de l'ensemble du masque 2.

En référence notamment à la figure 2, cet embout 6 comprend une embase 6₁, à partir de laquelle s'étend un fût cylindrique 6₂, destiné à être emmanché dans le col 4₂.

Cette embase est en outre munie d'une collerette 6₃, destinée à prendre appui contre l'extrémité libre 4₂₁ du col 4₂, cette extrémité libre formant ainsi un siège pour l'embout 6.

On notera par ailleurs que le fût 6_{2/} dont les dimensions axiales correspondent sensiblement à celles du col 4₂, définit une chambre (espace de prélèvement) notée 6₄. Du côté extérieur, opposé au volume V, il est prévu un raccord fileté 6₆, débouchant dans cette chambre 6₄, propre à coopérer par vissage avec un tuyau souple 12. Ce dernier est lui-même mis en communication avec un appareil 14, de type connu en soi, qui est propre à déterminer en temps réel au moins un paramètre respiratoire du patient. Cet appareil 14 est par ailleurs relié, par l'intermédiaire d'une ligne 16, à une alarme 18, qui est par exemple de type sonore.

Toujours en référence à la figure 2, l'embout 6 comprend à partir de l'embase 6₁, un fût cylindrique 6₇, destiné à être emmanché dans le col 5₁ de la poche réservoir en plastique souple désigné dans son ensemble par la référence 5.

Par ailleurs le fût 6₇ dont les dimensions axiales correspondent sensiblement à celles du col 5₁, définit une chambre notée 6₈.

Du côté extérieur, opposé au volume V, il est prévu un conduit 6₅ débouchant dans cette chambre 6₈. Ce conduit 6₅ est destiné à être raccordé avec une ligne 8, visible sur la figure 1, qui est mise en communication avec des moyens de fourniture d'Oxygène, notés 10, qui sont connus en tant que tels.

En revenant en particulier à la figure 2, le raccord fileté 6₆ débouche dans la chambre 6₄, s'étendant depuis l'embase 6₁ en direction du volume intérieur V. Il est à noter que cette chambre 6₄, qui est propre à prélever le CO2 expiré par le patient, débouche directement dans le raccord 6₆, en ce sens qu'il n'est pas mis en communication avec la chambre 6₈, elle-même protégée par une valve anti-retour désignée dans son ensemble par la référence 1, et fixée sur la partie 6₉ coté vers le volume intérieur V.

De plus, comme le montrent notamment les figures 1 et 2, l'alimentation en Oxygène par la partie 6₉ est positionnée de façon spéciale et expérimentalement vérifiée, par rapport au conduit de prélèvement 6₄;

**Cette** configuration relative des conduits de prélèvement et d'alimentation, est avantageuse, puisqu'elle permet d'éviter tout mélange entre les flux respectifs, formés par l'Oxygène et les gaz expirés par le patient.

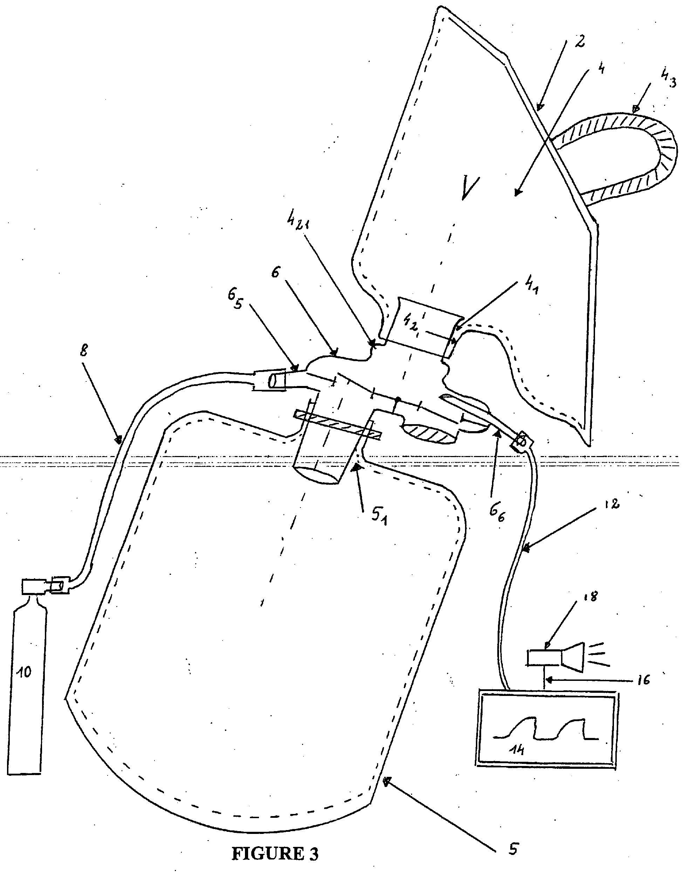

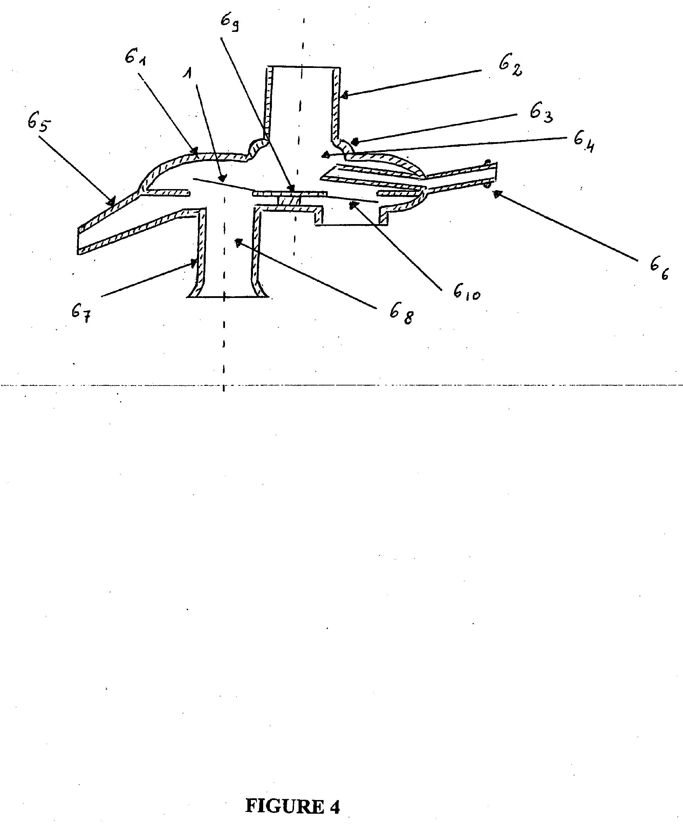

**A** titre de variante, dont une est représentée par les figures 3 et 4, la partie 6₉ d'alimentation en Oxygène pourrait être raccourcie, voire supprimé. De même, les pièces constitutives de ce système, peuvent être, en variante, monobloc ou assemblées.

La variante représentée par les figures 3 et 4, **avec un embout du deuxième type (B),** inclut dans **cet** embout 6 la valve d'expiration 6₁₀, qui remplace les valves normalement inclues latéralement dans le corps des masques, et destinée à évacuer les gaz expirés non prélevés pour le monitorage capnographique. Ceci présente l'avantage de mieux protéger les patients des risques de ré-inhalation du CO2 expiré, car la valve est intérieure (et donc moins vulnérable), et la valve est mieux orientée par rapport au flux expiré.

L'utilisation de l'ensemble de surveillance respiratoire, décrit ci-dessus, va maintenant être explicitée.

Il s'agit tout d'abord d'administrer de l'Oxygène au patient, de façon concentrée classique. Cette opération est réalisée grâce aux moyens de fourniture 10, qui délivrent, via la ligne 8 et le conduit 6₅, cet Oxygène vers la poche réservoir 5, elle-même en communication avec le volume intérieur V.

De façon concomitante, le CO2 expiré par le patient est dirigé vers l'appareil d'analyse 14, par l'intermédiaire successivement du conduit de prélèvement 6₄ du raccord 6₆ et du tuyau souple 12. Cet appareil 14 est notamment pourvu d'un calculateur propre à déterminer, entre autres, la fréquence respiratoire du patient, ainsi que le taux de dioxyde de carbone CO₂ présent dans le gaz expiré par ce patient.

Si les valeurs instantanées ainsi déterminées se situent en dehors d'une plage prédéterminée, l'alarme 18 est activée, ce qui permet au personnel soignant d'intervenir sur le patient. A titre d'exemple non limitatif, cette alarme 18 est activée si la fréquence respiratoire descend au-dessous de 6 par minute et/ou si le taux de CO₂ dans le gaz expiré devient supérieur à 30%.

L'invention permet d'atteindre les objectifs précédemment mentionnés. En effet, elle garantit une surveillance satisfaisante du patient, en particulier dans la mesure où elle permet de détecter toutes les variations réelles des paramètres respiratoires de ce dernier. Par conséquent, les risques d'accident se trouvent sensiblement éliminés.

De plus, l'invention confère une grande précision à la détermination des paramètres respiratoires du patient. A cet égard, il est tout particulièrement avantageux de disposer, sur un même embout, à la fois les moyens d'arrivée d'Oxygène et les moyens de prélèvement du CO2 expiré par le patient.

**Les revendications ci-après, décrivent les caractéristiques détaillées de l'invention, en référence aux** **figures 1** **et** **2** **pour la variante type (A), et de la même façon aux** **figures 3** **et** **4** **pour la variante type (B), les** **figures 2** **ou** **4** **représentant les deux types d'embouts, et les** **figures 1** **ou** **3** **représentant les montages correspondants sur les masques.**

## Revendications

1. Embout **(6) qui s'adapte sur un** masque à Oxygène "haute concentration" en ventilation spontanée **(2) propre à être solidarisé par rapport au corps (4) de ce masque (2), et par rapport à une poche réservoir d'oxygène (5), cet embout (6) comprenant des moyens (6₅) de connexion, propres à être raccordés à des moyens (10) de fourniture d'Oxygène, et qui permet en plus de mesurer de façon optimale les caractéristiques du CO2 expiré, caractérisé :**
- **en ce que** cet embout **(6)** comprend également des moyens **(6₆, 6₄)** efficaces de prélèvement du CO2 expiré par le patient (pour permettre son analyse séparément du 02 inspiré) ces moyens de prélèvement étant propres à être raccordés à un appareil **(14)** de détermination d'au moins un paramètre respiratoire du patient,
- **en ce que** les moyens de connexion **(6₅)** et les moyens de prélèvement **(6₆, 6₄)** s'étendent à partir d'une embase **(6₁)** dudit embout **(6),** cette embase étant propre à venir en appui contre un siège **(4₂₁)** du corps **(4)** du masque,
- **en ce que** les moyens de prélèvement comprennent une chambre de prélèvement **(6₄)**, s'étendant à partir de l'embase **(6₁)** en direction d'un volume intérieur **(V)** du corps **(4)** du masque, ainsi qu'un raccord de prélèvement **(6₆)** s'étendant à partir de l'embase **(6₁)** à l'opposé dudit volume intérieur **(V),** ce raccord de prélèvement débouchant directement dans la chambre de prélèvement,
- et **en ce que** l'embout **(6)** comprend un fût **(6₂)** propre à prendre appui contre des parois d'un col **(4₂)** du corps **(4)** du masque, et une partie intérieure **(6₉)** de séparation entre la chambre de prélèvement CO2 **(6₄)** et la chambre d'alimentation en Oxygène **(6₈),** vis-à-vis du fût **(6₂)** en direction du volume intérieur **(v)**, la chambre d'alimentation **(6₈)** étant séparée de la chambre de prélèvement **(6₄)** par une valve anti-retour (1).

2. Embout **qui s'adapte sur un** masque à **Oxygène** "haute concentration" **en ventilation spontanée** selon la revendication 1, **caractérisé en ce que** le raccord de prélèvement **(6₆)** est disposé à proximité d'un conduit **(6₅)** de connexion aux dits moyens de fourniture d'**Oxygène (10).**

3. Embout **qui s'adapte sur un** masque à **Oxygène** "haute concentration" **en ventilation spontanée** selon l'une quelconque des revendications 1 ou 2, **caractérisé en ce que** la partie intérieure de la chambre de prélèvement **(6₉)** est positionnée **de** façon **spéciale** par rapport au conduit de prélèvement **(6₄).**

4. Masque à **Oxygène** "haute concentration" **en ventilation spontanée (2)** comprenant un corps **(4)** destiné à recouvrir le nez et la bouche d'un patient, **et une poche réservoir en plastique souple (5) propre à être solidarisé par rapport à l'embout (6) notamment par emmanchement, ainsi que l'ajout d'un embout (6) propre à être solidarisé par rapport au corps (4), notamment par emmanchement, caractérisé en ce que l'embout (6) est conforme à l'une quelconque des revendications précédentes.**

5. Ensemble de surveillance respiratoire comprenant :
- un masque à **Oxygène** "haute concentration" **en ventilation spontanée (2)** conforme à la revendication 4 précédente ;
- des moyens **(10)** de fourniture d'**oxygène ;**
- des moyens **(8)** de raccordement entre ces moyens de fourniture **d'oxygène (10)** et les moyens de connexion **(6₅)** appartenant au masque **(2) ;**
- des moyens **(14)** de détermination d'au moins un paramètre respiratoire du patient ; et
- des moyens **(12)** de raccordement entre ces moyens de détermination **(14)** et les moyens de prélèvement **(6₆, 6₄)** appartenant au masque **(2).**

6. Ensemble de surveillance respiratoire selon la revendication 5, **caractérisé en ce que** cet ensemble comprend également une alarme **(18)** propre à être activée si une valeur du ou de chaque paramètre respiratoire, déterminée par les moyens de détermination **(14)**, se situe en dehors d'une plage prédéterminée.

7. Ensemble de surveillance respiratoire selon la revendication 5 ou 6, **caractérisé en ce qu'**un paramètre respiratoire est la fréquence respiratoire du patient.

8. Ensemble de surveillance respiratoire selon l'une quelconque des revendications 5 à 7, **caractérisé en ce qu'**un paramètre respiratoire est le taux de dioxyde de carbone (CO₂) présent dans le gaz expiré par le patient.

9. Ensemble de surveillance respiratoire selon l'une quelconque des revendications 5 à 8, **caractérisé en ce que** l'embout du masque a une configuration permettant des mesures capnographiques.

## Patentansprüche

1. Anschlussstück (6) für eine Maske für "hochkonzentrierten" Sauerstoff bei spontaner Atmung (2), das sich am Gehäuse (4) dieser Maske (2) und an einer Sauerstofftasche(5) befestigen lässt, wobei dieses Anschlussstück (6) Verbindungsmittel (6₅) beinhaltet, die sich an Mittel (10) für die Sauerstoffzufuhr anschließen lassen und es darüber hinaus eine optimale Messung der Charakteristiken des ausgeatmeten CO₂ ermöglicht, **dadurch gekennzeichnet:**
- **dass** dieses Anschlussstück (6) außerdem Mittel (6₆, 6₄) für eine effiziente Entnahme des vom Patienten ausgeatmeten CO₂ beinhaltet (um dessen, vom eingeatmeten O₂ getrennte, Analyse zu ermöglichen), wobei sich diese Entnahmemittel an einen Apparat (14) für die Bestimmung von mindestens einem Parameter der Atmung des Patienten anschließen lassen,
- **dass** die Verbindungsmittel (6₅) und die Entnahmemittel (6₆, 6,4) von einem Sockel (6₁) des genannten Anschlussstücks ausgehen, wobei dieser Sockel an einem Sitz (4₂₁) des Gehäuses (4) der Maske anliegt,
- **dass** die Entnahmemittel eine Entnahmekammer (6₄) beinhalten, die sich vom Sockel (6₁) aus in Richtung eines Innenraums (v) des Gehäuses der Maske (4) erstreckt, sowie einen Entnahmeanschluss (6₆), der sich von dem Sockel (6₁) gegenüber dem genannten Innenraum (V) erstreckt, wobei dieser Entnahmeanschluss direkt in der Entnahmekammer mündet,
- und **dass** das Anschlussstück (6) einen Schaft (6₂) beinhaltet, der an den Wandungen eines Halses (4₂) des Gehäuses (4) der Maske anliegt, und einen Innenteil (6₉) für die Trennung zwischen der Entnahmekammer für CO₂ (6₄) und der Kammer für die Versorgung mit Sauerstoff (6₈) gegenüber dem Schaft (6₂) in Richtung des Innenraums (V), wobei die Versorgungskammer (6₈) von der Entnahmekammer (6₄) anhand eines Rückschlagventils (1) getrennt ist.

2. Anschlussstück für eine Maske für "hochkonzentrierten" Sauerstoff bei spontaner Atmung nach Anspruch 1, **dadurch gekennzeichnet, dass** der Entnahmeanschluss (6₆) in der Nähe einer Leitung (6₅) für die Verbindung mit den genannten Mitteln für die Versorgung mit Sauerstoff (10) angeordnet ist.

3. Anschlussstück für eine Maske für "hochkonzentrierten" Sauerstoff bei spontaner Atmung nach einem der Ansprüche 1 oder 2, **dadurch gekennzeichnet, dass** der Innenteil der Entnahmekammer (6₉) auf besondere Weise im Verhältnis zur Entnahmeleitung (6₄) positioniert ist.

4. Maske für "hochkonzentrierten" Sauerstoff bei spontaner Atmung (2), bestehend aus einem Gehäuse (4) für die Abdeckung der Nase und des Mundes des Patienten und einer Vorratstasche aus flexiblem Kunststoff (5) für die Befestigung am Anschlussstück (6), insbesondere durch Aufstecken, sowie das Hinzufügen eines Anschlussstücks (6) für die Anbringung am Gehäuse (4), insbesondere durch Aufstecken, **dadurch gekennzeichnet, dass** das Anschlussstück (6) irgendeinem der voranstehenden Ansprüche entspricht.

5. Atmungsüberwachungsvorrichtung, bestehend aus:
- einer Maske für "hochkonzentrierten" Sauerstoff bei spontaner Atmung (2) gemäß dem voranstehenden Anspruch 4;
- Mitteln (10) für die Versorgung mit Sauerstoff;
- Mitteln (8) für die Verbindung zwischen diesen Mitteln für die Versorgung mit Sauerstoff (10) und den Verbindungsmitteln (6₅), die zur Maske (2) gehören;
- Mitteln (14) für die Bestimmung von mindestens einem Parameter der Atmung des Patienten;
- Mitteln (12) für den Anschluss zwischen diesen Bestimmungsmitteln (14) und den Entnahmemitteln (6₆, 6₄), die zur Maske (2) gehören.

6. Atmungsüberwachungsvorrichtung nach Anspruch 5, **dadurch gekennzeichnet, dass** diese Vorrichtung außerdem einen Alarm (18) beinhaltet, der aktiviert werden kann, wenn ein Wert jedes der Atmungsparameter, die anhand der Bestimmungsmittel (14) ermittelt werden, außerhalb eines vorgegebenen Bereichs liegt.

7. Atmungsüberwachungsvorrichtung nach Anspruch 5 oder 6, **dadurch gekennzeichnet, dass** ein Atmungsparameter die Atemfrequenz des Patienten ist.

8. Atmungsüberwachungsvorrichtung nach einem der Ansprüche 5 bis 7, **dadurch gekennzeichnet, dass** ein Atmungsparameter der Kohlendioxidgehalt (CO₂) in dem vom Patienten ausgeatmeten Gas ist.

9. Atmungsüberwachungsvorrichtung nach einem der Ansprüche 5 bis 8, **dadurch gekennzeichnet, dass** das Anschlussstück der Maske eine Konfiguration besitzt, die kapnographische Messungen ermöglicht.

## Claims

1. Nozzle (6) that fits on a "High Concentration" Oxygen mask in spontaneous breathing (2) that can be connected to the body (4) of this mask (2), and to an oxygen reserve bag (5), this nozzle (6) comprises connecting units (6₅), that can be connected to the oxygen supply units (10), and it also enables optimal measurement of the characteristics of the exhaled CO2, **characterized in that**:
- This nozzle (6) also comprises efficient sampling devices (6₆, 6₄) for the CO2 that is exhaled by the patient (to allow a CO2 analysis that is separate from the inhaled 02). These sampling devices can be connected to a determination device (14) for at least one respiratory parameter of the patient,
- The connecting units (6₅) and sampling devices (6₆, 6₄) extend from the base (6₁) of the said nozzle (6), this base is in contact against a seating device (4₂₁) of the body (4) of the mask,
- The sampling devices include a sampling chamber (6₄) that extends from the base (6₁) towards the interior volume (V) of the body (4) of the mask, and a sampling connector (6₆) that extends from the base (6₁) in the opposite direction of the said interior volume (V), this sampling connector opens directly onto the sampling chamber,
- And the nozzle (6) consists of a drum (6₂) that can be placed against the walls of the neck (4₂) of the body (4) of the mask, and a lower part (6₉) that separates the CO2 sampling chamber (64) and the Oxygen supply chamber (6₈), opposite the storage drum (6₂) in the direction of the interior volume (V), the supply chamber (6₈) is separated from the sampling chamber (6₄) by a non-return valve (1)

2. Nozzle that fits on a "High Concentration" Oxygen mask for spontaneous breathing as per claim 1, **characterized in that** the sampling connector (6₆) is placed near a connection duct (6₅) of the said Oxygen supply units (10).

3. Nozzle that fits on a "High Concentration" Oxygen mask for spontaneous breathing as per any of the previous claims, 1 or 2, **characterized in that** the lower part of the sampling chamber (6₉) is positioned in a special manner in relation to the sampling conduit (6₄).

4. "High Concentration" Oxygen mask for spontaneous breathing (2) comprising a body (4) designed to cover the nose and mouth of the patient, and a soft plastic bag (5) that can be fitted onto the nozzle (6) particularly by pressing, and also by adding a nozzle (6) that can be fitted to the body (4) particularly by pressing, **characterized in that** the nozzle (6) is in compliance with any of the previous claims.

5. Respiratory monitoring apparatus comprising:
- A "High Concentration" Oxygen mask for spontaneous breathing (2) in compliance with previous claim 4;
- Oxygen supply units (10);
- Connection units (8) between these Oxygen supply units (10) and the connecting units (6₅) of the mask (2) ;
- Determination devices (14) for at least one respiratory parameter of the patient; and
- Connection units (12) between these determination devices (14) and the sampling devices (6₆, 6₄) of the mask (2).

6. Respiratory monitoring apparatus as per claim 5, **characterized in that** this apparatus also contains an alarm (18) that can be activated if a value of each respiratory parameter, determined by the determination devices (14), falls outside a predetermined range.

7. Respiratory monitoring apparatus as per claim 5 or 6, **characterized in that** a respiratory parameter is the respiratory rate of the patient.

8. Respiratory monitoring apparatus as per any of the claims 5 to 7, **characterized in that** a respiratory parameter is the rate of carbon dioxide (CO₂) found in the gas exhaled by the patient.

9. Respiratory monitoring apparatus as per any of the claims 5 to 8, **characterized in that** the nozzle of the mask has a configuration that allows capnographic measurements.
